(19) Europäisches Patentamt
European Patent Office
Office européen des brevets

(11) Veröffentlichungsnummer: **0 033 928**
**B1**

# (12) EUROPÄISCHE PATENTSCHRIFT

(45) Veröffentlichungstag der Patentschrift: **08.05.85**

(51) Int. Cl.⁴: **C 11 B 9/00** // C07D317/12, A61K7/00

(21) Anmeldenummer: **81100715.2**

(22) Anmeldetag: **02.02.81**

(54) **Verwendung alkylsubstituierter 1,3-Dioxolane als Riechstoffe, sowie diese enthaltende Riechstoffkompositionen.**

(30) Priorität: **08.02.80 DE 3004661**

(43) Veröffentlichungstag der Anmeldung: **19.08.81 Patentblatt 81/33**

(45) Bekanntmachung des Hinweises auf die Patenterteilung: **08.05.85 Patentblatt 85/19**

(84) Benannte Vertragsstaaten: **CH DE FR GB LI NL**

(56) Entgegenhaltungen:
**FR - A - 2 147 091**
**FR - A - 2 225 102**
**US - A - 4 159 347**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Conrad, Jens, Dr., Dürerweg 15, D-4010 Hilden (DE)**
Erfinder: **Schaper, Ulf-Armin, Dr., Nixenstrasse 17, D-4000 Düsseldorf (DE)**
Erfinder: **Bruns, Klaus, Dr., Notburgaweg 6, D-4150 Krefeld-Traar (DE)**

**EP 0 033 928 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Es wurde gefunden, daß alkylsubstituierte 1,3-Dioxolane der allgemeinen Formel

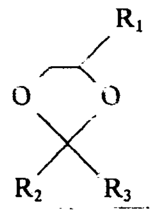

in der $R_1$ einen Alkylrest mit 4—12 Kohlenstoffatomen, $R_2$ Wasserstoff, einen Alkylrest mit 1—7 Kohlenstoffatomen, einen Cycloalkyl- oder Cycloalkenylrest mit 5—8 Kohlenstoffatomen, einen α-Furylrest oder einen gegebenenfalls alkylsubstituierten Arylrest, und $R_3$ Wasserstoff oder einen Alkylrest mit 1—3 Kohlenstoffatomen darstellen, in vorteilhafter Weise als Riechstoffe mit einer kräftigen, überwiegend fruchtigen Geruchsnote verwendet werden können.

Die Herstellung der erfindungsgemäß als Riechstoffe zu verwendenden alkylsubstituierten 1,3-Dioxolane erfolgt nach allgemein bekannten Verfahren der organischen Synthese. Ein Syntheseweg besteht in der Acetalisierung von Carbonylverbindungen mit den Resten $R_2$ und $R_3$ mit 1,2-Diolen der Kettenlänge $C_6—C_{14}$ in Gegenwart saurer Katalysatoren, unter Entfernen des Reaktionswassers mittels eines Lösungsmittels durch azeotrope Destillation oder durch Umsetzung des Reaktionswassers mit einem Orthoameisensäuretriester. Die Reaktion verläuft nach folgender Gleichung:

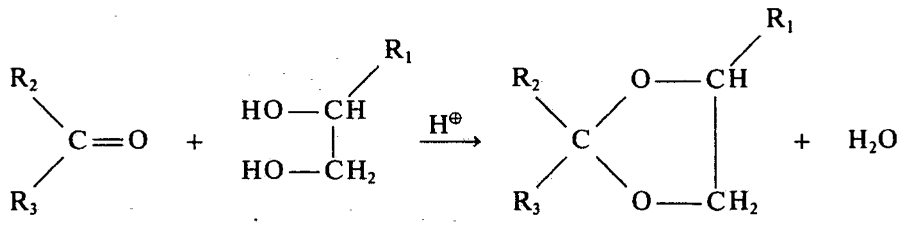


Darüber hinaus ist bereits eine größere Anzahl der erfindungsgemäß als Riechstoffe zu verwendenden alkylsubstituierten 1,3-Dioxolane nach speziellen Verfahren hergestellt worden, wie dies für 2,2-Dimethyl-4-dodecyl-1,3-dioxolan von R. P. Hanzlik und M. Leinwetter in J. Org. Chem. 43, 438 (1978), für 4-Heptyl, 4-Octyl-, 4-Decyl- und 4-Dodecyl-1,3-dioxolan von J. Rosenthal und D. Elad in J. Org. Chem. 33, 805 (1968), für 4-Octyl-1,3-dioxolan von R. Lalende et al in Tetrahedron Letters 1969, 745 und für 2,2-Dimethyl-4-pentyl-1,3-dioxolan von S. Raucher in Tetrahedron Letters 1976, 1161, für 2,2-Dimethyl-4-undecyl-1,3-dioxolan von H. Nakada in Agric. Biol. Chem. 41, 1761 (1977) und für 2-Methyl-4-hexyl-1,3-dioxolon von E. Tobler in Helv. Chim. Acta 52, 408 (1969) beschrieben wird. Keiner dieser Veröffentlichungen ist jedoch ein Hinweis auf Riechstoffeigenschaften zu entnehmen, noch daß derartige alkylsubstituierte 1,3-Dioxolane mit Vorteil als Riechstoffe in den verschiedenartigsten Riechstoffkompositionen eingesetzt werden können.

Aus der FR-A-2 147 091 sind spezielle Dioxolan-Derivate bekannt, die in 4-Stellung am Ring eine Carbonestergruppe besitzen. Es handelt sich dabei nicht um Riechstoffe, die für Parfümierungszwecke eingesetzt werden, sondern um Substanzen, die den Geschmack von Lebensmitteln verbessern oder verändern sollen. Die Hydrolyseempfindlichkeit der Carbonestergruppen verhindert im übrigen den Einsatz derartiger Verbindungen unter stärker sauren oder alkalischen Bedingungen.

In der US-A-4 159 347 sind Dioxolanderivate beschrieben, die am Ring in 4-Stellung durch Wasserstoff oder Methyl substituiert sind und in 2-Stellung einen einfach oder mehrfach ungesättigten aliphatischen Rest aufweisen. Auch diese Verbindungen sind keine Riechstoffe, sondern dienen der Hervorhebung und Steigerung des Aromas von Nahrungsmitteln, Kaugummis, Zahnpasten und medizinischen Produkten. Die Verbindungen weisen zudem infolge ihres ungesättigten Charakters eine gewisse Oxidationsempfindlichkeit auf, die ihren Einsatz in feinverteilten, pulverförmigen oder sonstwie in besonderem Maße der Luft ausgesetzten Produkten problematisch macht.

Als erfindungsgemäß zu verwendende alkylsubstituierte 1,3-Dioxolane sind z. B.

4-Hexyl-, 4-Octyl-, 4-Decyl-, 2-Methyl-4-hexyl-, 2-Methyl-4-octyl-, 2-Methyl-4-decyl-,
2-Ethyl-4-octyl-, 2-Isopropyl-4-decyl-, 2-Isopropyl-4-dodecyl-, 2-Pentyl-4-hexyl-, 2-Heptyl-4-butyl,
2,2-Dimethyl-4-hexyl-, 2,2-Dimethyl-4-octyl-, 2-Methyl-2-ethyl-4-butyl-, 2-Methyl-2-ethyl-4-hexyl-,
2-Cyclohexyl-4-butyl-, 2-Cyclohex-3-enyl-4-butyl-, 2-Cyclooctyl-4-butyl-, 2-α-Furyl-4-butyl-,
2-Cyclohex-3-enyl-4-hexyl-, 2-Phenyl-4-hexyl-, 2-Phenyl-4-octyl-, 2-Isobutyl-4-butyl-,
2-Isobutyl-4-hexyl-, 2-Isobutyl-4-octyl-, 2-(2-Pentyl)-4-butyl-, 2-(2-Pentyl)-4-hexyl-1,3-dioxolan

zu nennen.

Die erfindungsgemäß zu verwendenden alkylsubstituierten 1,3-Dioxolane stellen wertvolle Riechstoffe mit charakteristischen, interessanten Duftnoten dar. Ein besonderer Vorteil ist ihre sehr gute

Kombinationsfähigkeit zu neuartigen Geruchsnuancen.

Die erfindungsgemäß zu verwendenden alkylsubstituierten 1,3-Dioxolane können mit anderen Riechstoffen in den verschiedensten Mengenverhältnissen zu neuen Riechstoffkompositionen gemischt werden. Der Anteil der erfindungsgemäß zu verwendenden alkylsubstituierten 1,3-Dioxolane in den Riechstoffkompositionen wird in den Mengen von 1 bis 50 Gewichtsprozent, bezogen auf die gesamte Komposition, bewegen. Derartige Kompositionen könen direkt als Parfüm oder auch zur Parfümierung von Kosmetika wie Cremes, Lotionen, Duftwässern, Aerosolen, Mundpflegemitteln, Toiletteseifen usw. dienen. Sie können aber auch zur Geruchsverbesserung technischer Produkte wie Wasch- und Reinigungsmittel, Desinfektionsmittel, Textilbehandlungsmittel usw. eingesetzt werden.

Die nachfolgenden Beispiele sollen den Gegenstand der Erfindung näher erläutern, ohne ihn jedoch hierauf zu beschränken.

## Beispiele

Zunächst wird eine allgemeine Arbeitsvorschrift angegeben, nach der die in nachstehender Tabelle aufgeführten, erfindungsgemäß als Riechstoffe zu verwendenden alkylsubstituierten 1,3-Dioxolane hergestellt wurden.

0,1 Mol der entsprechenden Carbonylverbindung, 0,1 Mol des entsprechenden 1,2-Alkandiols, 0,1 Mol Orthoameisensäuretriethylester und 0,5 g p-Toluolsulfonsäure wurden 1 Stunde lang bei Raumtemperatur verrührt. Danach wurde langsam ein Gemisch aus Ameisensäureethylester und Ethanol abdestilliert. Der abgekühlte Rückstand wurde in Ether aufgenommen, mit Sodalösung und Wasser gewaschen, über Natriumsulfat getrocknet, eingeengt und im Vakuum destilliert.

Tabelle

| Produkt | Geruchsnote | Siedepunkt °C | Druck mbar | Brechungsindex $n_D^{20}$ |
|---|---|---|---|---|
| 4-Hexyl-1,3-dioxolan | bittere Nuß-Note | 42 | 0,02 | 1,4335 |
| 4-Octyl-1,3-dioxolan | Fettaldehyd-Note | | | 1,4381 |
| 4-Decyl-1,3-dioxolan | frische Geranylacetat-Note | 90 | 0,02 | 1,4430 |
| 2-Methyl-4-hexyl-1,3-dioxolan | fruchtig, krautig | | | 1,4292 |
| 2-Methyl-4-octyl-1,3-dioxolan | Liebstock-Note | 69 | 0,02 | 1,4348 |
| 2-Methyl-4-decyl-1,3-dioxolan | Walnuß-Jasmon-Note | 80 | 0,02 | 1,4396 |
| 2-Ethyl-4-octyl-1,3-dioxolan | Liebstock-, Walnußnote | 78 | 0,02 | 1,4342 |
| 2-Isopropyl-4-decyl-1,3-dioxolan | süße Johannisbrot-Note | 135 | 0,65 | 1,4423 |
| 2-Isopropyl-4-dodecyl-1,3-dioxolan | Johannisbrot-Note | 155 | 0,65 | 1,4462 |
| 2-Pentyl-4-hexyl-1,3-dioxolan | blumig, grün | 75 | 0,02 | 1,4389 |
| 2-Heptyl-4-butyl-1,3-dioxolan | Nußnote, süß | 140 | 20 | 1,4396 |
| 2,2-Dimethyl-4-hexyl-1,3-dioxolan | Jasmon-Note | 50 | 0,15 | 1,4251 |
| 2,2-Dimethyl-4-octyl-1,3-dioxolan | frische Wäsche-Note | 122 | 20 | 1,4322 |
| 2-Methyl-2-ethyl-4-butyl-1,3-dioxolan | fruchtig, krautig | 73 | 16 | 1,4240 |
| 2 Methyl-2-ethyl-4-hexyl-1,3-dioxolan | würzig | 105 | 20 | 1,4308 |

Tabelle (Fortsetzung)

| Produkt | Geruchsnote | Siedepunkt °C | Druck mbar | Brechungsindex $n_D^{20}$ |
|---|---|---|---|---|
| 2-Cyclohexyl-4-butyl-1,3-dioxolan | fruchtig | 54 | 0,06 | 1,4602 |
| 2-Cyclohex-3-enyl-4-butyl-1,3-dioxolan | fruchtig, Himbeer-, Erdbeer-Note | 70 | 0,03 | 1,4702 |
| 2-Cyclooctyl-4-butyl-1,3-dioxolan | süß | 91 | 0,02 | 1,4728 |
| 2-α-Furyl-4-butyl-1,3-dioxolan | Jasmon-, Pilz-Note | 63 | 0,02 | 1,4708 |
| 2-Cyclohex-3-enyl-4-hexyl-1,3-dioxolan | Himbeer-, Erdbeer-Note | 90 | 0,02 | 1,4699 |
| 2-Phenyl-4-hexyl-1,3-dioxolan | blumig, süß, Tabak-Note | 104 | 0,06 | 1,4952 |
| 2-Phenyl-4-octyl-1,3-dioxolan | Fettalkohol-Note | 118 | 0,02 | 1,4920 |
| 2-Isobutyl-4-butyl-1,3-dioxolan | Ananas-Note | 96 | 23 | 1,4295 |
| 2-Isobutyl-4-hexyl-1,3-dioxolan | herb-fruchtig | 125 | 23 | 1,4350 |
| 2-Isobutyl-4-octyl-1,3-dioxolan | Blaubeer-Note | 154 | 21 | 1,4395 |
| 2-(2-Pentyl)-4-butyl-1,3-dioxolan | süß | 109 | 21 | 1,4335 |
| 2-(2-Pentyl)-4-hexyl-1,3-dioxolan | Erdbeer-Note | 100 | 7 | 1,4385 |

Frucht-Komplex

| | |
|---|---|
| 2-Cyclohex-3-enyl-4-butyl-1,3-dioxolan | 300 Gew.-Teile |
| Phenylethylalkohol | 130 Gew.-Teile |
| Benzylacetat | 100 Gew.-Teile |
| Linalylacetat | 50 Gew.-Teile |
| Citronellol | 50 Gew.-Teile |
| Citronenöl | 50 Gew.-Teile |
| Orangenöl, süß | 50 Gew.-Teile |
| Cyclovertal, 10%ig | 50 Gew.-Teile |
| Phenylethylacetat | 40 Gew.-Teile |
| Aldehyd C18 (Nonalacton) 10%ig | 40 Gew.-Teile |
| Galaxolid | 30 Gew.-Teile |
| Aurantesin | 30 Gew.-Teile |
| Benzylpropionat | 30 Gew.-Teile |
| Aldehyd C14 (Undecalacton) 10%ig | 20 Gew.-Teile |
| Phenylisobutyrat | 20 Gew.-Teile |
| Cyclamenaldehyd | 10 Gew.-Teile |
| | 1000 Gew.-Teile |

Jasmin-Komplex

| | |
|---|---|
| 2,2-Dimethyl-4-hexyl-1,3-dioxolan | 100 Gew.-Teile |
| Linalool | 350 Gew.-Teile |
| Benzylacetat | 100 Gew.-Teile |
| Lyral | 100 Gew.-Teile |
| Dimethylbenzylcarbinylacetat | 50 Gew.-Teile |
| Benzylsalicylat | 50 Gew.-Teile |
| Ethyllinalool | 50 Gew.-Teile |
| Benzylalkohol | 40 Gew.-Teile |
| Ylang-Ylang-Öl | 40 Gew.-Teile |

| | |
|---|---|
| Benzylphenylacetat | 20 Gew.-Teile |
| Citronellol | 20 Gew.-Teile |
| Benzylbenzoat | 20 Gew.-Teile |
| Cyclamenaldehyd | 15 Gew.-Teile |
| α-Amylzimtaldehyd | 15 Gew.-Teile |
| Methylnaphthylketon | 10 Gew.-Teile |
| Nerol | 10 Gew.-Teile |
| Methyleugenol | 6 Gew.-Teile |
| Indoflor | 1 Gew.-Teile |
| Hexenylbenzoat | 1 Gew.-Teile |
| Methylheptenon 20%ig | 1 Gew.-Teile |
| Decanol 10%ig | 1 Gew.-Teile |
| | 1000 Gew.-Teile |

## Patentansprüche

1. Verwendung von alkylsubstituierten 1,3-Dioxolanen der allgemeinen Formel

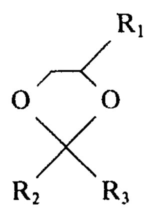

in der $R_1$ einen Alkylrest mit 4—12 Kohlenstoffatomen, $R_2$ Wasserstoff, einen Alkylrest mit 1—7 Kohlenstoffatomen, einen Cycloalkyl- oder Cycloalkenylrest mit 5—8 Kohlenstoffatomen, einen α-Furylrest oder einen gegebenenfalls alkylsubstituierten Arylrest, und $R_3$ Wasserstoff oder einen Alkylrest mit 1—3 Kohlenstoffatomen darstellen, als Riechstoffe.

2. Riechstoffkompositionen, dadurch gekennzeichnet, daß sie alkylsubstituierte 1,3-Dioxolane der in Anspruch 1 angegebenen Formel in einer Menge von 1 bis 50 Gewichtsprozent, bezogen auf die gesamte Komposition, enthalten.

## Claims

1. The use of alkyl-substituted 1,3-dioxolanes corresponding to the following general formula

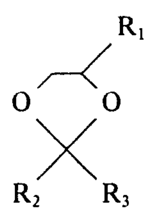

in wich $R_1$ is a $C_4—C_{12}$ alkyl radical, $R_2$ is hydrogen, a $C_1—C_7$ alkyl radical, a $C_5—C_8$ cycloalkyl or cycloalkenyl radical, an α-furyl radical or an optionally alkyl-substituted aryl radical and $R_3$ is hydrogen or a $C_1—C_3$ alkyl radical, as perfumes,

2. Perfume compositions, characterized in that they contain alkyl-substituted 1,3-dioxolanes corresponding to the formula in Claim 1 in a quantity of from 1 to 50% by weight, based on the composition as a whole.

## Revendications

1. Utilisation comme substances odoriférantes des 1,3-dioxolanes alcoyle substitués de formule générale:

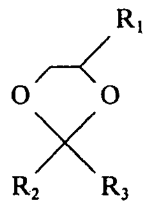

où $R_1$ est un reste alcoyle comportant 4 à 12 atomes de carbone, $R_2$ un hydrogène, un reste alcoyle à 1 à 7 atomes de carbone, un reste cycloalcoyle ou cycloalkényl à 5 à 8 atomes de carbone, un reste α-furyl ou un reste aryl éventuellement alcoyle substitué, et $R_3$, un hydrogène ou un reste alcoyle à 1 à 3 atomes de carbone.

2. Compositions odoriférantes caractérisées en ce qu'elles contiennent des 1,3-dioxolanes alcoyle-substitués répondant à la formule indiquée dans la revendication 1 dans une proportion de 1 à 50% en poids calculé sur le poids total de la composition.